# EUROPEAN PATENT APPLICATION

(11) **EP 4 806 388 A1**
(43) Date of publication of application: **16.09.2026**
(21) Application number: 24889019.6
(22) Date of filing: 29.10.2024
(51) Int. Cl.: A61C 19/04, A61B 5/00, A61C 9/00, G06T 1/00, A61B 18/20

(54) **INTRAORAL SCANNER**

(30) Priority: 06.11.2023 KR 20230152204
(71) Applicant: Arcreal Inc., Seoul 06180 (KR)
(72) Inventor: JEON, Seung Hyun, Seoul 06180 (KR); KIM, Kyong Kook, Seoul 06180 (KR); KANG, Seok Bong, Seoul 06180 (KR)
(74) Representative: Studio Torta S.p.A.
(86) International application number: PCT/KR2024/016708
(87) International publication number: WO 2025/100831

(57) **Abstract**

The present intraoral scanner includes: a light source unit configured to emit light; a first optical system configured to reflect light emitted from the light source unit to be moved along a first optical path; a second optical system configured to reflect light reflected from the first optical system toward an object; and a third optical system forming a pair of second optical paths with the second optical system, the third optical system being configured to reflect light, which is reflected from the object to the second optical system and moved along the pair of second optical paths, to an image sensor unit. The third optical system includes a pair of first reflectors forming the pair of second optical paths, a pair of second reflectors configured to reflect light reflected from the pair of first reflectors to the image sensor unit, and a spacing frame configured to support the pair of first and second reflectors in a state of being spaced apart.

## Description

### [Technical Field]

The present disclosure relates to an intraoral scanner, and more particularly, to an intraoral scanner configured to acquire a three-dimensional (3D) image of a subject's oral cavity.

### [Background Art]

Generally, an impression-taking procedure is performed during a diagnosis or treatment process for a dental patient. Impression-taking is a clinical procedure necessary for establishing a diagnosis and treatment plan for a patient by reflecting the state of teeth and tissues in the oral cavity onto an impression material. Recently, as digital technology has been applied to dental clinical and laboratory processes, there has been an increasing use of digital impressions, which involve scanning the inside of the oral cavity or an impression body and converting it into digital data without using impression materials. As the importance of digital impressions in dental diagnosis and treatment increases, technical developments for intraoral scanners are being actively pursued.

An intraoral scanner is a device or system that is inserted into the oral cavity of a dental patient to scan the 3D structure of teeth in a non-contact manner. In general, recently developed intraoral scanners capture two-dimensional (2D) image data of the oral cavity and perform 3D modeling of the oral structure based on the 2D image data. The intraoral scanner having such functions has an expanded range of clinical applications and can be used not only for tooth restoration treatments but also for the fabrication of implants, orthodontic appliances, and the like.

Meanwhile, the accuracy of an impression is crucial for successful dental treatment. Since a digital impression through an intraoral scanner does not suffer from deformation issues caused by the contraction or expansion of impression materials, it offers higher impression accuracy compared to traditional impression-taking methods. However, for an intraoral scanner to continue being used as a sophisticated dental procedure tool, it is necessary to improve the accuracy of scanning. Furthermore, since the intraoral scanner is used by being inserted into the oral cavity of a dental patient in a non-contact manner, it is desirable for the scanner to have a structure that ensures the patient feels comfortable during its use

### [Disclosure]

### [Technical Problem]

An aspect of the present disclosure provides an intraoral scanner in which a plurality of optical systems are arranged to have a structure suitable for being inserted into the oral cavity of a dental patient in a non-contact manner.

An aspect of the present disclosure provides an intraoral scanner with an improved structure.

### [Technical Solution]

An intraoral scanner according to an idea of the present disclosure includes: a light source unit configured to emit light; a first optical system configured to reflect light emitted from the light source unit to be moved along a first optical path; a second optical system configured to reflect light reflected from the first optical system toward an object; and a third optical system forming a pair of second optical paths with the second optical system, the third optical system being configured to reflect light, which is reflected from the object to the second optical system and moved along the pair of second optical paths, to an image sensor unit . The third optical system includes: a pair of first reflectors forming the pair of second optical paths; a pair of second reflectors configured to reflect light reflected from the pair of first reflectors to the image sensor unit; and a spacing frame configured to support the pair of first and second reflectors in a state of being spaced apart.

The pair of first and second reflectors may be configured to be respectively seated on the spacing frame.

The pair of first and second reflectors each include a pair of first and second reflecting surfaces that are configured to at least partially reflect light and are in contact with the spacing frame.

The spacing frame may include first and second spacing parts on an outside and inside thereof, respectively, on which the first and second reflecting surfaces are respectively supported.

The first spacing part includes a first outer support part and a first inner support part on which the first and second reflecting surfaces are respectively supported , and the second spacing part includes a second outer support part and a second inner support part on which the first and second reflecting surfaces are respectively supported , and the first outer support part, the first inner support part, the second outer support part, and the second inner support part may be configured to be spaced apart from each other by a predetermined angle.

The first inner support part and the second inner support part may be formed to be spaced apart at an angle larger than an angle formed between the first outer support part and the second outer support part.

The first outer support part and the second outer support part may be disposed such that centers thereof are on the same line as centers of the first inner support part and the second inner support part.

The spacing frame may include an upper spacing part and a lower spacing part respectively supporting an upper portion and a lower portion of the pair of first and second reflectors.

The upper spacing part and the lower spacing part may be configured to be spaced apart from each other by a predetermined distance.

The pair of first and second reflectors, together with the upper spacing part and the lower spacing part, may form a light passage through which light reflected by the third optical system passes.

The intraoral scanner may further include: a lens assembly having at least one lens and disposed on a third optical path formed between the pair of second reflectors and the image sensor unit; and a support frame on which the lens assembly is seated.

The support frame may include first and second support surfaces configured to support symmetrical portions of the lens assembly in a width direction while supporting the lens assembly.

The first and second support surfaces may be configured to spread at a predetermined angle.

The support frame may be integrally formed with the spacing frame.

The intraoral scanner may further include: a sensor frame on which the image sensor unit is disposed ; and a connecting member configured to connect the image sensor unit to the sensor frame.

A plurality of connecting members may be provided, and the plurality of connecting members may be disposed along a circumference of the image sensor unit and configured such that the image sensor unit is supported by the sensor frame.

The plurality of connecting members may connect the image sensor unit to be spaced apart from the sensor frame so as to form a separation space between the image sensor unit and the sensor frame.

The plurality of connecting members may be configured to independently adjust a position of the image sensor unit with respect to the sensor frame.

The position of the image sensor unit may include at least one of an angle and a spacing distance with respect to the sensor frame.

### [Advantageous Effects]

According to an aspect of the present disclosure, optical systems inside the intraoral scanner can be densely arranged at optimal positions.

According to an aspect of the present disclosure, by miniaturizing the intraoral scanner, it is easy to not only insert the scanner into the oral cavity of a dental patient but also move or change direction in the oral cavity, so that tooth scanning can be precisely performed.

According to an aspect of the present disclosure, a plurality of stereo images can be acquired through a single image sensor unit, thereby reducing the manufacturing cost of the intraoral scanner and achieving optimization of internal configurations and spatial utilization.

According to an aspect of the present disclosure, by configuring the arrangement between the optical systems to be maintained, deformation over time can be minimized.

### [Description of Drawings]

FIGS. 1 and 2 are perspective views of an intraoral scanner according to an embodiment of the present disclosure.
FIG. 3 is a schematic diagram showing a configuration in which an intraoral scanner according to an embodiment of the present disclosure is connected to an oral 3D modeling and visualization server.
FIGS. 4, 5, and 6 are views for illustrating an optical system of an intraoral scanner according to an embodiment of the present disclosure.
FIG. 7 is a view showing an example of stereo images acquired by an intraoral scanner according to an embodiment of the present disclosure.
FIG. 8 is a cross-sectional view of an intraoral scanner according to an embodiment of the present disclosure.
FIG. 9 is a view showing some configurations including a first optical system and a light source unit of an intraoral scanner according to an embodiment of the present disclosure.
FIG. 10 is a view showing a frame and adjacent configurations of an intraoral scanner according to an embodiment of the present disclosure.
FIG. 11 is a view showing a light emission unit and a first optical system of an intraoral scanner according to an embodiment of the present disclosure.
FIG. 12 is a view showing a third optical system of an intraoral scanner according to an embodiment of the present disclosure.
FIG. 13 is a view showing a lens assembly of an intraoral scanner according to an embodiment of the present disclosure.
FIGS. 14 to 16 are views showing an air flow of an intraoral scanner according to an embodiment of the present disclosure.

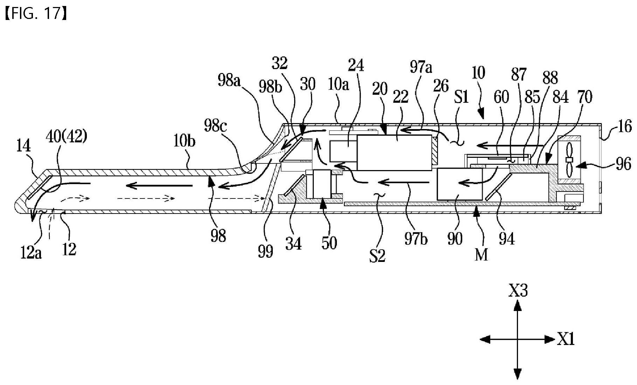

FIG. 17 is a view showing an air flow of an intraoral scanner according to another embodiment of the present disclosure.

### [Modes of the Invention]

The embodiments described in the present specification and the configurations shown in the drawings are merely preferred examples of the disclosed invention, and there may be various modifications capable of replacing the embodiments and drawings of the present specification at the time of filing the present application.

Also, the same reference numbers or signs presented in each drawing of the present specification denote parts or components that perform substantially the same functions.

Also, the terms used in the present specification are used for the purpose of describing the embodiments and are not intended to limit and/or restrict the disclosed invention. Singular expressions include plural expressions unless the context clearly indicates otherwise. In the present specification, terms such as "comprise" or "have" are intended to designate that features, numbers, steps, operations, components, parts, or combinations thereof described in the specification exist, and do not preclude the possibility of the presence or addition of one or more other features, numbers, steps, operations, components, parts, or combinations thereof .

Also, terms including ordinal numbers such as "first", "second", etc., used in the present specification may be used to describe various components, but the components are not limited by the terms, and the terms are used only for the purpose of distinguishing one component from another component. For example, a first component may be referred to as a second component without departing from the scope of the rights of the present disclosure, and similarly, a second component may also be referred to as a first component. The term "and/or" includes a combination of a plurality of related described items or any item among a plurality of related described items.

Also, terms such as "-unit", "-device", "-block", "-member", "-module", etc., may mean a unit for processing at least one function or operation. For example, the terms may mean at least one hardware such as an FPGA (field-programmable gate array)/ASIC (application specific integrated circuit), at least one software stored in a memory, or at least one process processed by a processor.

Hereinafter, embodiments according to the present disclosure will be described in detail with reference to the accompanying drawings. However, the following drawings attached to the present specification illustrate preferred embodiments of the present disclosure and serve to further enhance the understanding of the technical ideas of the present disclosure together with the contents of the invention described above, and thus the present disclosure should not be interpreted as being limited only to the matters described in such drawings .

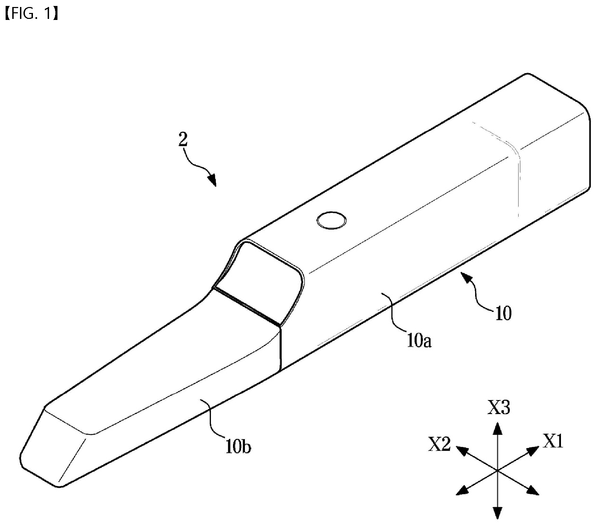

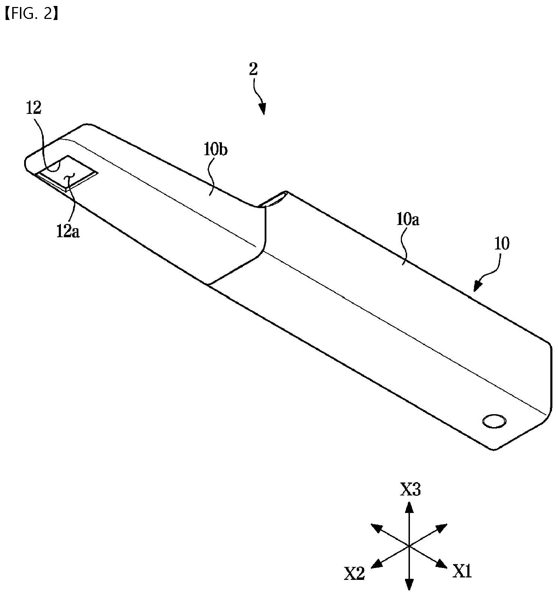

FIGS. 1 and 2 are perspective views of an intraoral scanner according to an embodiment of the present disclosure.

The intraoral scanner (2) may be configured to be gripped by a user, and may be configured such that at least a portion thereof can be inserted into the oral cavity while being gripped by the user.

The housing (10) of the intraoral scanner (2) forms an exterior of the intraoral scanner (2), and may be configured to accommodate a light source unit (20), a first optical system (30), a second optical system (40), a third optical system (50), and an image sensor unit (60), which will be described later, therein. As shown in FIGS. 1 and 2, the housing (10) may be formed in the shape of a long rod having a longitudinal direction in one direction, or may be formed in any shape suitable for being inserted into the oral cavity. Also, although the cross-section of the intraoral scanner (2) is illustrated as being substantially rectangular, it is not limited thereto, and the cross-section may be configured to include at least one of a circular shape, a streamlined shape, and a polygonal shape. In addition, when the cross-section of the housing (10) is formed in a polygonal shape, it may be configured to be rounded for a user's grip.

The housing (10) may include a first housing body (10a) and a second housing body (10b). The light source unit (20), the first optical system (30), the third optical system (50), and the image sensor unit (60) may be disposed inside the first housing body (10a), and the second optical system (40) may be disposed inside the second housing body (10b). The second housing body (10b) may be provided to be detachable from the first housing body (10a). When using the intraoral scanner (2), at least a portion of the intraoral scanner (2) may be inserted into the oral cavity of a patient. Through such a configuration, after using the intraoral scanner (2), the second housing body (10b) located at the front of the intraoral scanner may be separated from the first housing body (10a) to be separately sterilized or washed.

An opening forming part (12) may be formed adjacent to one end of the housing (10). Specifically, the opening forming part (12) is disposed adjacent to one end of the housing (10) to form an opening (12a) through which the inside and outside of the housing (10) are connected to each other.

The opening forming part (12) may be configured such that light generated or reflected inside the housing (10) is emitted to the outside through the opening (12a), and light from the outside can be introduced into the housing (10). In one embodiment, when the intraoral scanner (2) is inserted into the oral cavity, the opening (12a) may be configured to face a tooth for which scanning is required

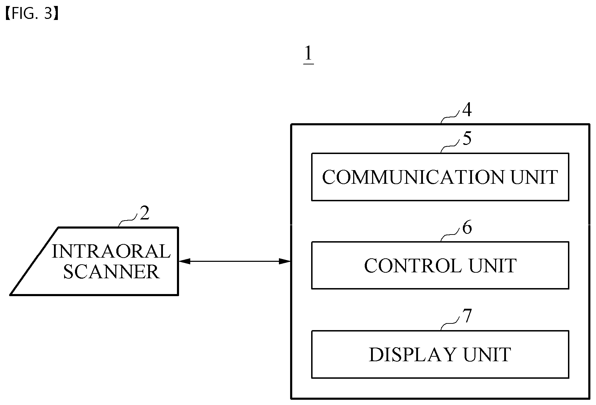

FIG. 3 is a schematic diagram showing a configuration of an intraoral scanning system in which an intraoral scanner is connected to an oral 3D modeling and visualization server according to an embodiment of the present disclosure. As illustrated, the intraoral scanning system (1) may include an intraoral scanner (2) capable of scanning a three-dimensional (3D) structure inside the oral cavity of a dental patient, and an oral 3D modeling and visualization server (4) connected to the intraoral scanner (2).

The intraoral scanner (2) may be inserted into the oral cavity by a user for example, dental medical staff to scan teeth in a non-contact manner, thereby capturing a plurality of two-dimensional (2D) image data. Also, the intraoral scanner (2) may transmit the plurality of captured 2D image data to the 3D modeling and visualization server (4), or may independently perform 3D oral structure modeling based on the 2D image data.

The intraoral scanner (2) may be connected to the 3D modeling and visualization server (4) through a network configured to enable wired or wireless communication. Here, depending on the installation environment, the network may include a wired network such as an electrical connection line like a copper cable, Ethernet, Power Line Communication (PLC), a telephone line communication device, and RS-serial communication; a wireless network such as a mobile communication network, Wireless LAN (WLAN), Wi-Fi, Bluetooth, and ZigBee; or a combination thereof.

The intraoral scanner (2) may exchange information and/or data, such as 2D image data and 3D oral structure model data, with the 3D modeling and visualization server (4). The intraoral scanner (2) and the 3D modeling and visualization server (4) may be configured to be physically separated as shown, but the configuration is not limited thereto. For example, the intraoral scanner (2) and the 3D modeling and visualization server (4) may be integrally configured within a single computing device.

The 3D modeling and visualization server (4) may perform 3D oral structure modeling based on at least two 2D image data or stereo images obtained from the intraoral scanner (2). To perform such functions, the 3D modeling and visualization server (4) may correspond to a computing device including a processor (e.g., CPU, GPU, AP, NPU, etc.) capable of performing image processing and 3D modeling, and a memory capable of storing 2D image data or 3D oral structure model data. In one embodiment, as illustrated, the 3D modeling and visualization server (4) may include a communication unit (5), a control unit (6), and a display unit (7). The communication unit (5) may be configured to transmit and receive information and/or data to and from the intraoral scanner (2). Specifically, the communication unit (5) may transmit a command signal from the control unit (6) to the intraoral scanner (2) and may receive image information of a target oral structure from the intraoral scanner (2) .

The control unit (6) may control the intraoral scanner (2) to capture an image of the target oral structure. Specifically, the control unit (6) may control at least one light source unit (20) (e.g., 220 of FIG. 2) installed inside the intraoral scanner (2) to emit light toward at least one of a plurality of optical systems. Also, the control unit (6) may control an image sensor unit (60) installed inside the intraoral scanner (2) to detect light reflected by at least one of the plurality of optical systems. The control unit (6) may control the image sensor unit (60) (e.g., 270 of FIG. 2) to acquire at least two or more stereo images from the detected image of light. The control unit (6) may control the display unit (7) to display the two or more stereo images received from the intraoral scanner (2). Alternatively or additionally, the control unit (6) may visualize 3D oral structure model data calculated based on the two or more stereo images and control it to be displayed on the display unit (7).

The display unit (7) may display information and/or data received from the intraoral scanner (2) or the control unit (6). In this case, the data displayed on the display unit (7) may include two stereo images or an image of a 3D oral structure model. In one embodiment, the display unit (7) may include a display panel device such as an LED display, an OLED display, an LCD display, a touch display, or the like.

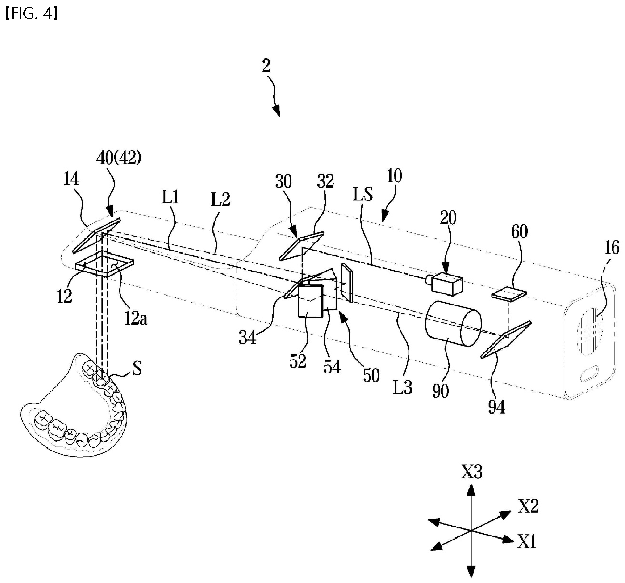

FIGS. 4, 5, and 6 are views for illustrating an optical system of an intraoral scanner according to an embodiment of the present disclosure. FIGS. 4, 5, and 6 are views provided to describe the internal configuration of the intraoral scanner (2).

The intraoral scanner (2) may include a housing (10), a light source unit (20), a first optical system (30), a second optical system (40), a third optical system (50), and an image sensor unit (60).

The light source unit (20) may be configured to generate light. The light generated by the light source unit (20) may be configured to pass through the optical systems. In this case, the light emitted from the light source unit (20) may correspond to patterned light or structured light. The light pattern may be a linear pattern, a dot pattern, or a pattern of an arbitrary shape. When the patterned light is emitted onto an object (S) such as a tooth in the oral cavity, a deformation of the pattern may occur according to the 3D structure of the surface of the object (S). Accordingly, the 3D structure of the object (S) can be identified and modeled based on information on the deformation of the pattern projected onto the surface of the object (S) or the change in the position of feature points.

The light source unit (20) may be disposed inside the housing (10). Specifically, the light source unit (20) may be configured to emit light toward a first optical system (30), which will be described later. An opening (12a) or the second optical system (40) may be disposed adjacent to one end of the housing (10), and the light source unit (20) may be disposed adjacent to the other end of the housing (10). Also, the light source unit (20) may be disposed at an arbitrary intermediate point between the one end and the other end of the housing (10). The arrangement of the light source unit (20) is not limited, and it is satisfied as long as the light generated by the light source unit (20) is emitted toward the first optical system (30) or the second optical system (40), preferably toward the first optical system (30).

The first optical system (30) may be configured to reflect the light emitted from the light source unit (20) toward the second optical system (40). The first optical system (30) may reflect light emitted along an emission axis (LS) so as to move along a first optical path (L1) spaced apart in a width direction (X2) or the width direction (X2) and a height direction (X3). The width direction (X2) may mean a direction related to the horizontal width perpendicular to the longitudinal direction (X1) of the intraoral scanner (2). The height direction (X3) may mean a direction related to the vertical height perpendicular to the longitudinal direction (X1) of the intraoral scanner (2). The width direction (X2) may be defined as a left-right direction, and the height direction (X3) may also be defined as an up-down direction. For convenience of description, the longitudinal direction (X1) may be defined as a first direction, the width direction (X2) as a second direction, and the height direction (X3) as a third direction.

The first optical system (30) may include a first reflector (32) and a second reflector (34). The first reflector (32) may be configured to reflect the light emitted from the light source unit (20) toward the second reflector (34). The first reflector (32) may be disposed at an inner upper portion of the housing (10). Also, the second reflector (34) may be configured to reflect the light reflected by the first reflector (32) toward the second optical system (40). The second reflector (34) may be disposed to be spaced apart from the first reflector (32).

The light emitted from the light source unit (20) may be reflected toward the second optical system (40) via the first reflector (32) and the second reflector (34) of the first optical system (30). The first reflector (32) of the first optical system (30) may form an emission axis (LS) with the light source unit (20), and the second reflector (34) of the first optical system (30) may form a first optical path (L1) with the second optical system (40) . The optical path refers to an area through which light passes or a central axis of a path along which light travels in space, and may also be defined as an optical axis.

The light source unit (20) is disposed to be spaced apart from the first optical path (L1), and the first and second reflectors (32, 34) may be configured to be inclined so that light emitted from the light source unit (20) can be moved to the second optical system (40) along the set first optical path (L1) . The inclined arrangement of the first and second reflectors (32, 34) will be described later.

The second optical system (40) reflects the light emitted from the second reflector (34) of the first optical system (30) toward the object (S), and the light reflected from the object (S) may be reflected by the second optical system (40) to travel toward the third optical system (50).

The second optical system (40) may include at least one reflector. This may be defined as a third reflector (42). For example, the second optical system (40) may be at least one mirror. In one embodiment, the second optical system (40) may be disposed at or around the opening (12a). For example, the second optical system (40) may be fixedly disposed on an inner side surface of the housing (10) adjacent to the opening (12a).

The third optical system (50) may be configured to reflect the light reflected from the second optical system (40) from the outside to the image sensor unit (60). Specifically, the third optical system (50) may reflect light reflected by the second optical system (40) from the object (S) toward the image sensor unit (60). The third optical system (50) may include one or more reflectors or mirrors for reflecting light.

The first to third optical systems (30, 40, 50) may be arranged to be aligned along a reference line (AL) passing through the first optical path (L1) as shown in FIGS. 4 to 6. The reference line may also be defined as a reference axis. As the first to third optical systems (30, 40, 50) are aligned along the reference line (AL), the travel distance of light can be minimized. Through this, light dispersed while passing through the optical systems can be minimized, thereby minimizing chromatic aberration, and clear and high contrast can be obtained even to the periphery of the object, so that optical performance can be improved.

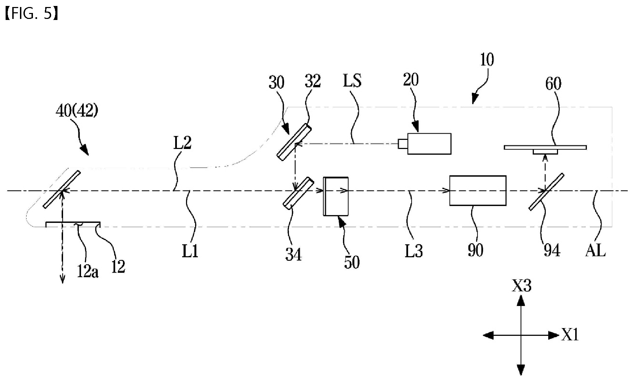

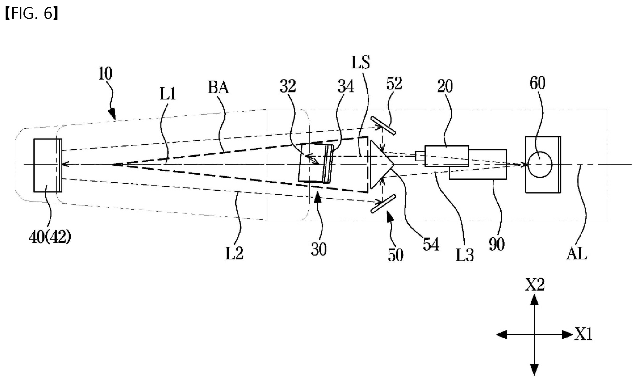

Also, the first to third optical systems (30, 40, 50) may be disposed on one virtual plane through which the reference line (AL) passes. The reference line (AL) may mean an extension line extending from the first optical path (L1). FIG. 5 may be a cross-sectional view of the intraoral scanner based on the corresponding plane. The image sensor unit (60) may also be disposed on the plane. The virtual plane is disposed so that the reference line (AL) of FIGS. 5 and 6 passes through it, and the plane may mean a plane extending in the height direction from the reference line (AL). In the present embodiment, the plane may be configured to pass through the center of the intraoral scanner (2).

The third optical system (50) may be disposed adjacent to the second reflector (34) of the first optical system (30). Specifically, the third optical system (50) may be disposed adjacent to the other surface of a reflection surface on which light is reflected in the second reflector (34) of the first optical system (30).

The third optical system (50) may include a fourth reflector (52) and a fifth reflector (54). A pair of fourth reflectors (52) may be provided, and the pair of fourth reflectors (52) may reflect the light reflected from the second optical system (40) toward the fifth reflector (54). The second optical system (40) may form a pair of second optical paths (L2) with the third optical system (50). Specifically, the pair of second optical paths (L2) may be formed by the second optical system (40) and the pair of fourth reflectors (52) of the third optical system (50). Since the pair of fourth reflectors (52) are configured to be spaced apart from each other, the pair of second optical paths (L2) may be configured to spread apart from each other in the width direction (X2). The pair of second optical paths (L2) may be formed to be identical in the height direction as shown in FIG. 5, but the present disclosure is not limited thereto. A central line of the pair of second optical paths (L2) may also be disposed parallel to the first optical path (L1).

The second reflector (34) of the first optical system (30) may be disposed between the pair of fourth reflectors (52). Specifically, the second optical system (40) and the pair of fourth reflectors (52) may form a blind area (BA) as shown in FIG. 6, and the second reflector (34) of the first optical system (30) may be disposed in the blind area (BA). Through this, the second reflector (34) of the first optical system (30) may be disposed so as not to interfere with light reflected from the second optical system (40) toward the pair of fourth reflectors (52) of the third optical system (50). Through such a configuration, the first optical path (L1) and the pair of second optical paths (L2) may not interfere with each other, and components inside the housing (10) can be more densely arranged.

A pair of fourth reflectors (52) may form a gap therebetween. By forming the gap between the pair of fourth reflectors (52), light reflected by the fourth reflectors (52) and then reflected the fifth reflectors (54) may pass through the gap to reach the image sensor unit (60). Here, the position and direction of each of the pair of fourth reflectors (52) may be set such that two images of the object (S), which are respectively reflected by two reflecting surfaces of the fifth reflectors (54) and detected by the image sensor unit (60), do not overlap each other and each of the images is entirely visible .

A pair of fifth reflectors (54) may be provided, and the pair of fifth reflectors (54) may be configured to reflect the light reflected from the pair of fourth reflectors (52) toward the image sensor unit (60). Specifically, the pair of fifth reflectors (54) may respectively reflect the light reflected from the pair of fourth reflectors (52) toward the image sensor unit (60). The light reflected from the pair of fifth reflectors (54) may pass through the gap formed between the pair of fourth reflectors (52) to reach the image sensor unit (60). Here, the position and direction of each of the pair of fifth reflectors (54) may be set such that two images of the object (S), which are respectively reflected by the fifth reflectors (54) and detected by the image sensor unit (60), do not overlap each other and each of the images is entirely visible.

The pair of fifth reflectors (54) may be connected to each other at one edge of each reflector. For example, the pair of fifth reflectors (54) may be configured in the form of a triangular prism disposed adjacently as shown in the drawings.

In one embodiment, each of the first optical system (30), the second optical system (40), or the third optical system (50) may be fixedly disposed at a predetermined position inside the housing (10). In this case, a driving unit for adjusting an angle of the first optical system (30), the second optical system (40), or the third optical system (50) may not be installed inside the housing (10). As such, since there is no need to dispose other electronic or mechanical components in the area where the first optical system (30), the second optical system (40), and the third optical system (50) are disposed inside the housing (10), the components inside the housing (10) can be densely arranged. Accordingly, since an optimal structure of the housing (10) can be designed from the dense structure of the first optical system (30), the second optical system (40), and the third optical system (50), an intraoral scanner (2) that has a small volume and is free in scanning operations in the oral cavity can be implemented .

In one embodiment, the angle formed between the pair of fourth reflectors (52) corresponds to a minor angle, that is, an angle smaller than 180 degrees, and the angle formed between the pair of fifth reflectors (54) may correspond to a major angle, that is, an angle larger than 180 degrees.

The image sensor unit (60) may be configured to detect light reflected from the third optical system (50). Specifically, the image sensor unit (60) may be configured to detect light that is reflected from the third optical system (50) and passes through the lens assembly (90) and the sensor reflector (94). In one embodiment, the image sensor unit (60) may be configured to acquire two stereo images from the light reflected from the third optical system (50). Specifically, the image sensor unit (60) may simultaneously acquire images of two lights respectively reflected by the fifth reflectors (54) of the third optical system (50) . As described above, the intraoral scanner (2) can acquire two stereo images using only one image sensor unit (60) through the third optical system (50). The two stereo images acquired from the image sensor unit (60) can be subsequently used for 3D oral structure modeling executed by a processor.

In the present embodiment, the fourth and fifth reflectors (52, 54) have been described as each having one reflecting surface, but the fourth and fifth reflectors (52, 54) may include two or more reflecting surfaces (n). In this case, the image sensor unit (60) may acquire 2n stereo images.

The image sensor unit (60) may be disposed inside the housing (10). Specifically, the image sensor unit (60) may be disposed adjacent to the other end of the housing (10). The image sensor unit (60) may be configured such that lights reflected from the fifth reflectors (54) of the third optical system (50) are incident thereon.

Through such configurations, light emitted from the light source unit (20) may be reflected toward the second optical system (40) by the first reflector (32) and the second reflector (34) of the first optical system (30). The light reflected from the second reflector (34) may be reflected toward the object (S) located outside the housing (10) through the second optical system (40) and the opening (12a). In addition, light reflected from the object (S) may be reflected by the second optical system (40) toward the fourth reflectors (52) of the third optical system (50). The light reflected by the fourth reflectors (52) may be reflected by the fifth reflectors (54) toward the image sensor unit (60).

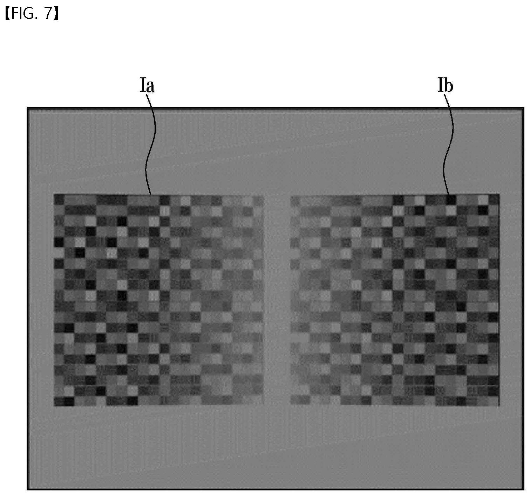

FIG. 7 is a view showing an example of stereo images acquired by an intraoral scanner according to an embodiment of the present disclosure.

FIG. 7 is a view showing an example of stereo images acquired according to an embodiment of the present disclosure. The image sensor unit (60) may be configured to detect light reflected from the third optical system (50). The image sensor unit (60) may be configured to acquire two stereo images (Ia, Ib) from the light reflected from the third optical system (50). Specifically, the image sensor unit (60) may simultaneously acquire two stereo images (Ia, Ib) respectively reflected by the pair of fifth reflectors (54) of the third optical system (50). Based on the two stereo images (Ia, Ib) obtained in this way, a processor may extract depth data and perform 3D modeling of an oral structure, which is the object (S), based on the depth data.

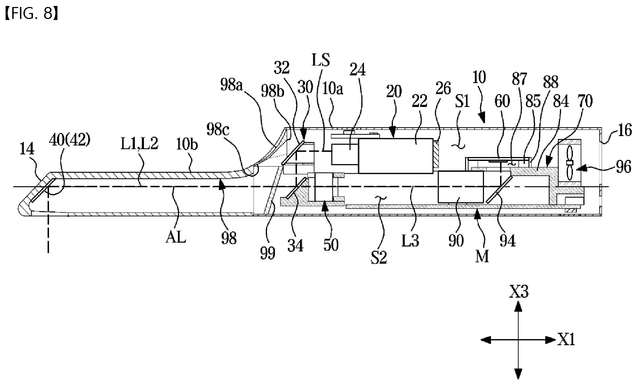

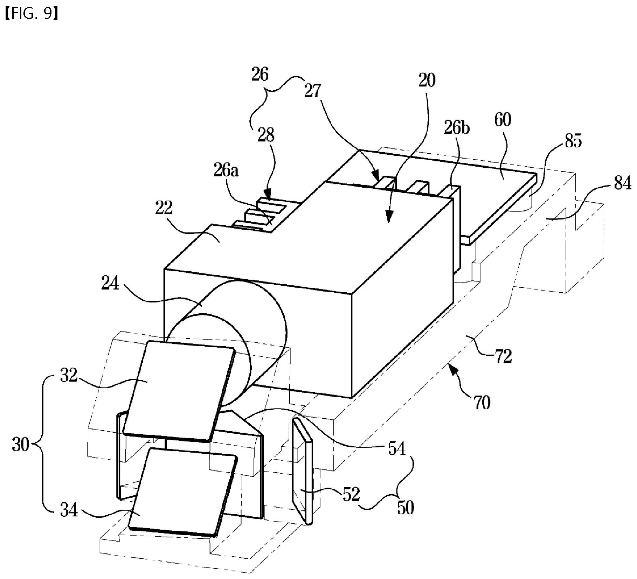

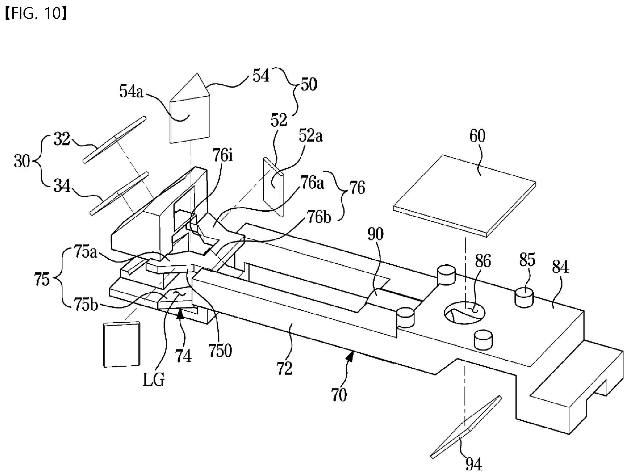

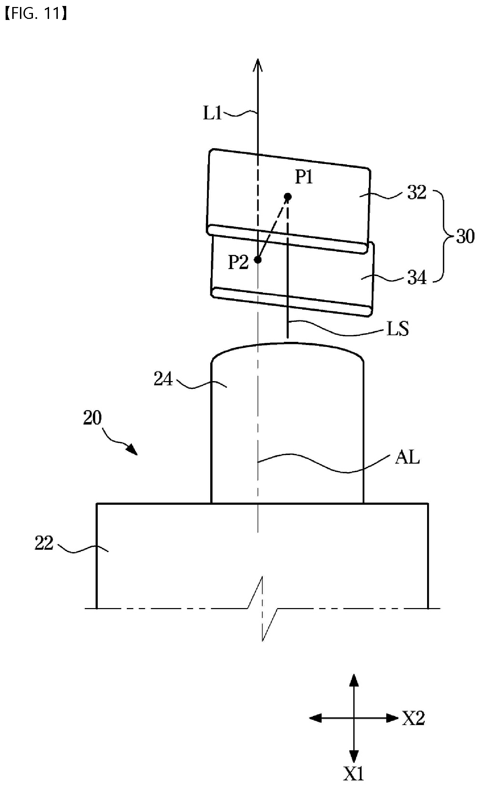

FIG. 8 is a cross-sectional view of an intraoral scanner according to an embodiment of the present disclosure, FIG. 9 is a view showing some configurations including a first optical system and a light source unit of an intraoral scanner according to an embodiment of the present disclosure, FIG. 10 is a view showing a frame and adjacent configurations of an intraoral scanner according to an embodiment of the present disclosure, and FIG. 11 is a view showing a light emission unit and a first optical system of an intraoral scanner according to an embodiment of the present disclosure .

FIGS. 8 to 11 describe specific configurations of an intraoral scanner in which the optical systems described above are disposed.

The intraoral scanner (2) may include a frame (70).

The frame (70) may be disposed inside the housing (10) so that components such as the light source unit (20) and the optical systems (30, 50) are disposed. The frame (70) may be disposed inside the housing (10) and configured to improve durability of the intraoral scanner (2) and protect components against external forces or shocks.

The first optical system (30), the third optical system (50), the light source unit (20), and the image sensor unit (60) may form one module by being mounted and installed on the frame (70). This may be defined as an optical module (M). The optical module (M) may be mounted on the first housing body (10a), and by managing the first optical system (30), the third optical system (50), the light source unit (20), and the image sensor unit (60) as one module, assembly of the intraoral scanner (2) can be made easier, improving productivity and space efficiency.

The frame (70) may include an inner frame (72). The inner frame (72) may be disposed in a direction parallel to a direction in which the first to third optical systems (30, 40, 50) are disposed. Specifically, the inner frame (72) may be disposed parallel to the reference line (AL).

The inner frame (72) may be configured to partition a first space (S1) and a second space (S2) of the housing (10). The first and second spaces (S1, S2) may be defined as an upper space and a lower space, respectively. The inner frame (72) may be disposed between the first and second spaces (S1, S2) and configured to support components of the intraoral scanner (2).

The light source unit (20) may be disposed at one side of the inner frame (72) as shown in FIG. 9.

Specifically, the light source unit (20) may be disposed to be located in the first space (S1), which is an upper space.

The light source unit (20) may include a light source body (22) and a light emission unit (24). Light may be generated inside the light source body (22), and the generated light may be emitted through the light emission unit (24). The light emission unit (24) will be described in detail later.

The light source unit (20) may include a heat dissipation unit (26).

The heat dissipation unit (26) may be in contact with the light source body (22) and configured to dissipate heat generated from the light source unit (20). The heat dissipation unit (26) may include a first heat dissipation member (27) configured to face a heat dissipation fan (96), which will be described later, and a second heat dissipation member (28) configured along an air flow direction of the heat dissipation fan (96).

The first and second heat dissipation members (27, 28) may include a base plate (26a) that is closely attached to the light source body (22) and heat dissipation plates (26b) extending from the base plate (26a) to widen a heat dissipation area for the internal space of the housing (10). The heat dissipation plate (26b) of the first heat dissipation member (27) may be configured to face the heat dissipation fan (96) to be described later, and the heat dissipation plate (26b) of the second heat dissipation member (28) may be disposed along the air flow direction by the heat dissipation fan (96) to be described later.

The first heat dissipation member (27) may be configured to perform heat dissipation through more direct heat exchange by external air flowing from the heat dissipation fan (96). The second heat dissipation member (28) may be configured along the flow direction of air by the heat dissipation fan (96) so that heat exchange can occur in proportion to an air flow rate or an air velocity.

The light emission unit (24) may be configured so that light is emitted along an emission axis (LS). Specifically, the light emission unit (24) may emit light to form the emission axis (LS) spaced apart from the first optical path (L1).

Specifically, the emission axis (LS) may be spaced apart from the first optical path (L1) in the width direction (X2) and the height direction (X3) (refer to FIGS. 8 and 11). To this end, the first optical system (30) may reflect light emitted along the emission axis (LS) to be moved along the first optical path (L1) spaced apart in the width direction (X2) and the height direction (X3).

The emission axis (LS) through which light emitted from the light emission unit (24) passes may be parallelly spaced apart from the first optical path (L1). However, the present disclosure is not limited thereto, and the emission axis (LS) may be formed to be inclined at a predetermined angle with respect to the first optical path (L1). Assuming a virtual plane in the height direction (X3) passing through the reference line (AL), the light emission unit (24) may be disposed to be spaced apart from the virtual plane. Also, the light emission unit (24) may be disposed so that the emission axis (LS) is spaced apart from the virtual plane in the second direction. The virtual plane may also be defined as a center plane.

The first optical system (30) may include first and second reflectors (32, 34). The first and second reflectors (32, 34) may be disposed to be spaced apart in the height direction (X3). Specifically, the first and second reflectors (32, 34) may be disposed such that points where the emission axis (LS) is reflected in the first and second reflectors (32, 34) and points forming the first optical path (L1) correspond to a spacing direction between the emission axis (LS) and the first optical path (L1). That is, when a point where the emission axis (LS) is reflected in the first reflector (32) is a first point (P1, refer to FIG. 11) and a point forming the first optical path (L1) in the second reflector (34) is a second point (P2, refer to FIG. 11), the first point (P1) may be formed to correspond to a spacing direction and a spacing distance of the emission axis (LS) and the first optical path (L1) with respect to the second point (P2). At this time, the first point (P1) formed on the first reflector (32) may be disposed to be spaced apart from the virtual plane. The first and second reflectors (32, 34) may be disposed so that the first and second points (P1, P2) can be formed on the first and second reflectors (32, 34), and their size or arrangement is not limited.

The first reflector (32) reflects light emitted along the emission axis (LS) from the light source unit (20) to the second reflector (34), and the second reflector (34) may reflect the light reflected from the first reflector (32) to the second optical system (40) along the first optical path (L1).

The first and second reflectors (32, 34) may be disposed to be inclined so that light emitted along the emission axis (LS) can move along the first optical path (L1) spaced apart from the emission axis (LS) in the second and third directions (X2, X3). Specifically, based on being disposed perpendicularly to the first direction (X1), the first and second reflectors (32, 34) may be disposed to be inclined in the second direction (X2) and the third direction (X3).

Referring to FIG. 11, since the emission axis (LS) is disposed on the right side of the first optical path (L1), the first and second reflectors (32, 34) may be configured to be inclined in a leftward direction and a downward direction. At this time, the first and second reflectors (32, 34) may be disposed to be inclined in the same direction and at the same angle. That is, the first and second reflectors (32, 34) may be disposed parallel to each other while being inclined so that light passing through the emission axis (LS) passes through the first optical path (L1).

Through this configuration, the first optical system (30) is not constrained by the arrangement of the light source unit (20), and even if the emission axis (LS) emitted from the light source unit (20) is spaced apart from the first optical path (L1) in the width direction (X2), the first optical path (L1) can be configured to pass through the center of the intraoral scanner (2).

Furthermore, by disposing the light source unit (20) without being constrained by its size, shape, or emission direction, spatial efficiency for the components inside the intraoral scanner (2) can be improved, and the configurations of the intraoral scanner (2) can be more densely arranged.

The second optical system (40) may be disposed to correspond to the opening (12a) of the housing (10). Light passing through the first optical path (L1) is emitted toward a tooth to be scanned through the second optical system (40) and the opening (12a), and the second optical system (40) may be disposed to correspond to the opening (12a) of the housing (10) in order to reflect light reflected from the tooth to the third optical system (50) .

The second optical system (40) may be disposed adjacent to one end of the housing (10). Specifically, the second optical system (40) may be disposed on an optical system installation part (14) formed inside the housing (10). The optical system installation part (14) is disposed adjacent to the opening (12a) and may support the second optical system (40) such that the second optical system (40) is disposed to be inclined with respect to the first optical path (L1). The optical system installation part (14) may also be defined as a second optical system installation part since the second optical system (40) is disposed thereon. The second optical system (40) may be configured to be fixed to the optical system installation part (14) by an adhesive member (not shown). The adhesive member may include an adhesive tape. By applying adhesive tape as the adhesive member, the thickness of the adhesive member can be minimized, and deformations such as thermal deformation or swelling over time can be minimized. However, the present disclosure is not limited thereto, and it is satisfied if an adhesive member having little thermal deformation and little deformation over time is applied.

The optical system installation part (14) is formed to be inclined at a predetermined angle from one direction, so that the cross-sectional area of the housing (10) can be formed to be smaller toward the front end. Through this, the intraoral scanner (2) can be more easily inserted into the oral cavity.

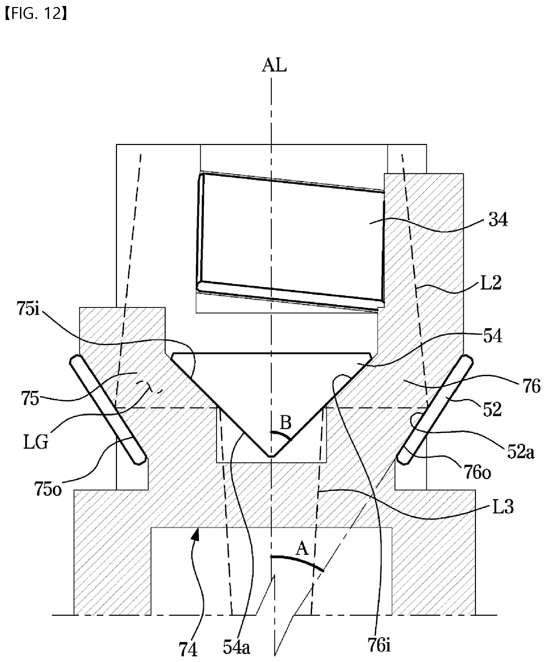

FIG. 12 is a view showing a third optical system of an intraoral scanner according to an embodiment of the present disclosure. The description will be made with reference to the preceding drawings.

The frame (70) may include a spacing frame (74) supporting the third optical system (50).

The spacing frame (74) may be configured to support the fourth reflector (52) and the fifth reflector (54) of the third optical system (50) in a state of being spaced apart. That is, the fourth and fifth reflectors (52, 54) may be configured to be respectively seated on the spacing frame (74). The spacing frame (74) supporting the fourth and fifth reflectors (52, 54) may be one component of the third optical system (50).

A pair of fourth reflectors (52) includes a pair of reflecting surfaces forming a pair of second optical paths (L2), and a pair of fifth reflectors (54) may include a pair of reflecting surfaces for reflecting light reflected by the fourth reflectors (52) to the image sensor unit (60). For convenience of description, the reflecting surface of the fourth reflector (52) may be defined as a first reflecting surface (52a), and the reflecting surface of the fifth reflector (54) may be defined as a second reflecting surface (54a). At least a portion of the first and second reflecting surfaces (52a, 54a) is configured to reflect light and may be configured to be in contact with the spacing frame (74).

The spacing frame (74) may include first and second spacing parts (75, 76) respectively supporting the first and second reflecting surfaces (52a, 54a) on the outside and inside thereof. The first spacing part (75) supports one of the fourth reflectors (52) and one of the fifth reflectors (54), and the second spacing part (76) may support the other fourth reflector (52) and the other fifth reflector (54).

The first and second spacing parts (75, 76) are configured to support the reflecting surfaces (52a, 54a) that reflect light in the fourth and fifth reflectors (52, 54), so that the spacing distance or spacing angle between the reflecting surfaces (52a, 54a) can be maintained, and even when deformation occurs in the fourth and fifth reflectors (52, 54) as they age over time, the spacing relationship can be maintained .

The first and second spacing parts (75, 76) are configured as a pair spaced apart vertically, so that the fourth and fifth reflectors (52, 54) can be stably supported. Specifically, the first spacing part (75) includes a first upper spacing part (75a, refer to FIG. 10) and a first lower spacing part (75b, refer to FIG. 10) spaced apart vertically, and the second spacing part (76) may include a second upper spacing part (76a, refer to FIG. 10) and a second lower spacing part (76b, refer to FIG. 10) spaced apart vertically. The first upper spacing part (75a) and the first lower spacing part (75b) may be configured to be spaced apart from each other by a predetermined distance. Also, the second upper spacing part (76a) and the second lower spacing part (76b) may also be configured to be spaced apart from each other by a predetermined distance. The intervals at which the upper spacing parts (75a, 76a) and the lower spacing parts (75b, 76b) are spaced apart may be formed to be the same.

The upper spacing parts (75a, 76a) and the lower spacing parts (75b, 76b), together with the fourth and fifth reflectors (52, 54), may form a light passage (LG, refer to FIGS. 10 and 12) of light moving along the second optical path (L2), and light moving along the pair of second optical paths (L2) may be configured to move only through the light passage (LG) . Through this configuration, noise light can be minimized, and the resolution of stereo images can be improved.

The first and second spacing parts (75, 76) may include outer support parts (75o, 76o) supporting the fourth reflectors (52) and inner support parts (75i, 76i) supporting the fifth reflectors (54), respectively. The centers of the outer support parts (75o, 76o) and the centers of the inner support parts (75i, 76i) may be disposed on the same line as the reference line (AL).

The outer support parts (75o, 76o) and the inner support parts (75i, 76i) may be configured to be spaced apart from each other by a predetermined angle. That is, through this configuration, the fourth reflector (52) supported by the outer support parts (75o, 76o) and the fifth reflector (54) supported by the inner support parts (75i, 76i) may be configured to be spaced apart by a predetermined angle. Specifically, based on the virtual reference line (AL), when an angle between the reference line (AL) and the outer support parts (75o, 76o) is A and an angle between the reference line (AL) and the inner support parts (75i) is B, it may be configured to form a relationship of A < B. Also, when the angle between the outer support parts (75o, 76o) is 2A and the angle between the inner support parts (75i) is 2B, the relationship of 2A < 2B can be satisfied.

Through this configuration, light reflected from the fifth reflector (54) can be prevented from being reflected back to the fourth reflector (52), and light moving along the pair of second optical paths (L2) can be moved to the image sensor unit (60) along the fourth and fifth reflectors (52, 54) without being overlapped.

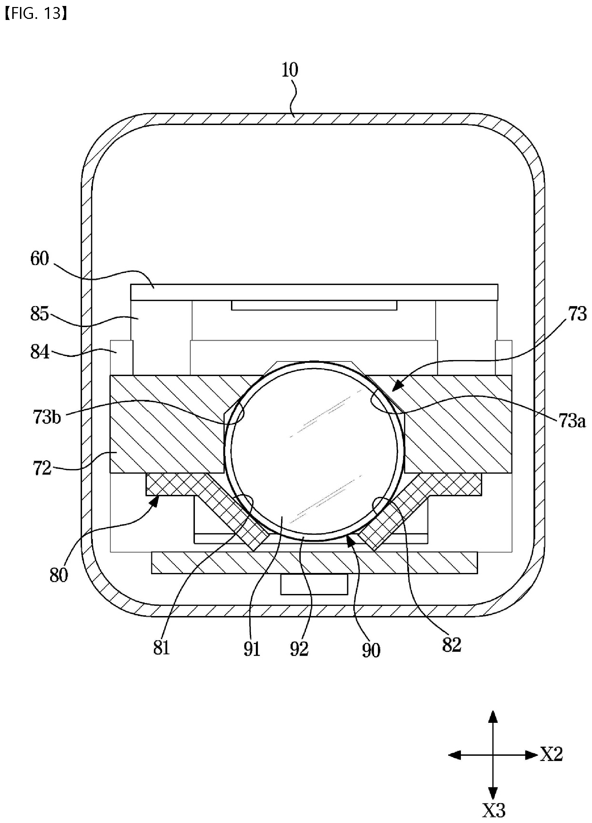

FIG. 13 is a view showing a lens assembly of an intraoral scanner according to an embodiment of the present disclosure.

The intraoral scanner (2) may include a lens assembly (90).

The lens assembly (90) may be configured to adjust the focus of light so that the light reflected through the fifth reflector (54) reaches the image sensor unit (60). The fifth reflector (54) and the image sensor unit (60) may form a third optical path (L3). The lens assembly (90) may be disposed on the third optical path (L3). The lens assembly (90) may include at least one lens (91) through which the third optical path (L3) passes, and a lens case (92) in which the lens (91) is accommodated. The lens case (92) may be formed in a substantially cylindrical shape, and its shape is not limited. It is satisfied if the lens case (92) is a case in which the lenses (91) through which the light passing through the third optical path (L3) passes are accommodated.

The frame (70) may include a support frame (80) on which the lens assembly (90) is seated.

The support frame (80) may be integrally formed with the spacing frame (74). Specifically, the support frame (80) may be integrally formed with the inner frame (72), and the spacing frame (74) may also be integrally formed with the inner frame (72). Since the support frame (80) is integrally formed with the spacing frame (74), the spacing distance and angle between the third optical system (50) and the lens assembly (90) can be kept constant. Through this, the light reflected from the third optical system (50) can be configured to constantly pass through the lens assembly (90), so that stable stereo images can be acquired from the image sensor unit (60).

The support frame (80) can prevent the lens assembly (90) from rotating in yaw, pitch, and roll directions, and can support the lens assembly (90) to be stably positioned.

The support frame (80) forms an accommodation space so that the lens assembly (90) can be seated therebetween and may include a support surface for supporting the lens assembly (90).

The support surface may be configured to support the lens assembly (90). The support surface may include a first support surface (81) and a second support surface (82). The first and second support surfaces (81, 82) may be configured to support symmetrical portions of the lens assembly (90) in the width direction (X2). The first support surface (81) supports at least a portion of the outer peripheral surface of the lens assembly (90), and the second support surface (82) may be formed to support at least a portion of the rest. The first and second support surfaces (81, 82) may be configured to be symmetrical to each other about a virtual plane, and the first and second support surfaces (81, 82) may be configured to be spaced apart from each other by a predetermined angle. As an example, the first and second support surfaces may be formed in a substantially V-shape, and the lens assembly (90) may be supported by the first and second support surfaces (81, 82) so that the third optical path (L3) can pass through the lens (91).

The inner frame (72) may include a mounting part (73) for restricting the movement of the lens assembly (90) seated on the support frame (80). The mounting part (73) may be formed concavely from the lower surface of the inner frame (72). Through this configuration, the pair of support surfaces can support the lower portion of the lens assembly (90), and the mounting part (73) can be configured to support the upper surface of the lens assembly (90).

The mounting part (73) includes mounting surfaces (73a, 73b) configured to contact at least a portion of the upper surface of the lens assembly (90), and can prevent the lens assembly (90) seated on the support surface from being detached and prevent positional change through the mounting surfaces (73a, 73b).

The intraoral scanner (2) may include a sensor reflector (94, refer to FIGS. 8 and 10). The sensor reflector (94) may be configured to reflect light, which passes through the lens assembly (90) and moves along the third optical path (L3), to the image sensor unit (60). The image sensor unit (60) may be disposed parallel to a direction in which the first to third optical systems (30, 40, 50) are disposed. The light passing through the lens assembly (90) is reflected by the sensor reflector (94), so that stereo images can be acquired in the image sensor unit (60) disposed parallel to the one direction.

The frame (70) may include a sensor frame (84) on which the image sensor unit (60) is disposed. The sensor frame (84) may be connected to the inner frame (72) and may be integrally formed with the inner frame (72). Since the sensor frame (84) is integrally formed with the inner frame (72), the spacing distance and angle between the third optical system (50) and the image sensor unit (60) can be kept constant. Through this, the light reflected from the third optical system (50) can constantly reach the image sensor unit (60), so that stable stereo images can be acquired from the image sensor unit (60).

The image sensor unit (60) may be disposed on the sensor frame (84). The frame (70) may also include a connecting member (85) so that the image sensor unit (60) can be connected to the sensor frame (84). The connecting member (85) may be a component of the sensor frame (84). The image sensor unit (60) may be supported by the sensor frame (84) through the connecting member (85).

The image sensor unit (60) and the sensor frame (84) may be disposed in directions parallel to each other. Specifically, the sensor frame (84) may be disposed parallel to the inner frame (72). Also, the image sensor unit (60) and the sensor frame (84) may be disposed in a direction parallel to a direction in which the first to third optical systems (30, 40, 50) are disposed.

The sensor frame (84) may include a sensor hole (86). The sensor hole (86) may be formed so that light reflected by the sensor reflector (94) reaches the image sensor unit (60) through the sensor frame (84). The sensor hole (86) may be formed so that the third optical path (L3) passes therethrough, and may be formed so that the image sensor unit (60) is exposed to the sensor reflector (94).

The image sensor unit (60) is disposed parallel to the sensor frame (84), but may be disposed to be spaced apart from the sensor frame (84) by a predetermined distance. Specifically, a separation space (87) having a predetermined distance may be formed between the image sensor unit (60) and the sensor frame (84) .

The connecting member (85) may connect the image sensor unit (60) to be disposed spaced apart from the sensor frame (84) so that the separation space (87) is formed between the image sensor unit (60) and the sensor frame (84).

A plurality of connecting members (85) may be provided, and the plurality of connecting members (85) may be disposed along the circumference of the image sensor unit (60). As in the present embodiment, the plurality of connecting members (85) may be disposed adjacent to the four corners of the image sensor unit (60) so that four connecting members (85) are provided. However, the number or arrangement of the connecting members (85) is not limited, and it is satisfied if it is a configuration connecting the image sensor unit (60) from the sensor frame (84).

The plurality of connecting members (85) may be configured to be able to independently adjust the position of the image sensor unit (60) with respect to the sensor frame (84). Specifically, the plurality of connecting members (85) may be configured so that the image sensor unit (60) has different distances from the sensor frame (84). The plurality of connecting members (85) may each be configured to independently adjust the spacing distance and may be configured to maintain the set spacing distance. Through this configuration, the image sensor unit (60) can be fine-adjusted by position with respect to the sensor frame (84), and the plurality of connecting members (85) can be configured to adjust at least one of an angle and a spacing distance of the image sensor unit (60) with respect to the sensor frame (84) .

The frame (70) may include a sensor guide (88). The sensor guide (88) may be one component of the sensor frame (84). The sensor guide (88) may be configured to be formed along the circumference of the image sensor unit (60). The sensor guide (88) may be formed along the circumference of the image sensor unit (60) so that the separation space (87) is not exposed. Since the sensor guide (88) is formed along the circumference of the image sensor unit (60), it can prevent light from the outside, which can be noise, from being emitted into the image sensor unit (60), and can further improve the quality of a stereo image formed through light moving along the third optical path (L3). The sensor guide (88) may be formed to have a height corresponding to a distance by which the image sensor unit (60) is spaced apart from the sensor frame (84).

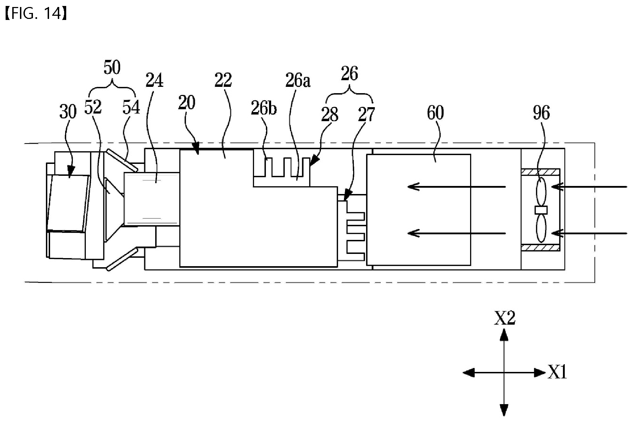

FIGS. 14 to 16 are views showing an air flow of an intraoral scanner according to an embodiment of the present disclosure.

The intraoral scanner (2) may include a heat dissipation fan (96).

The heat dissipation fan (96) may be disposed within the intraoral scanner (2) and configured to circulate external air to dissipate heat generated therein. The housing (10) includes an air hole (16), and the heat dissipation fan (96) may be provided to cause the air introduced through the air hole (16) to flow. The air introduced through the air hole (16) by the heat dissipation fan (96) may be discharged to the outside through the opening (12a) of the housing (10).

The air hole (16) may be provided at a rear side of the housing (10), and the heat dissipation fan (96) may be formed at an inner side of the rear of the housing (10) to correspond to the air hole (16). The heat dissipation fan (96) may be disposed adjacent to an inner surface of the housing (10) and configured to be able to circulate external air.

The heat dissipation fan (96) may be disposed at one end of the frame (70). Specifically, it may be configured to cause air to flow in one direction, which is an arrangement direction of the first to third optical systems (30, 40, 50). The optical systems (30, 40, 50) and the heat dissipation fan (96) may be arranged along the one direction in the order of the second optical system (40), the first optical system (30), the third optical system (50), and the heat dissipation fan (96).

The intraoral scanner (2) may include an air flow path (97). The air flow path (97) may be a flow path flowing above the inner frame (72). By causing air to flow through the air flow path (97), the heat dissipation fan (96) may dissipate heat generated from the light source unit (20) and the image sensor unit (60) located above the inner frame (72).

The intraoral scanner (2) may include a flow path guide (98).

The flow path guide (98) may be formed to guide the air flow so that external air introduced from the air hole (16) can be discharged through the opening (12a). The flow path guide (98) may be disposed adjacent to the first optical system (30). In the present embodiment, the flow path guide (98) may be disposed between the first optical system (30) and the second optical system (40). The flow path guide (98) may guide air, which is introduced and flows through the air hole (16) by the heat dissipation fan (96), to be discharged through the opening (12a).

The flow path guide (98) may be formed along the inner surface of the housing (10). The flow path guide (98) may be formed as a separate component, but in the present embodiment, it is formed along the inner surface of the housing (10) to guide the air flow inside the housing (10).

The flow path guide (98) includes a guide member (98a).

The guide member (98a) may be provided on a path of the air flow (97) and provided to guide air flowing inside the housing (10) toward the front of the housing where the opening is located. The guide member (98a) may be disposed between the second housing body (10b) and the first housing body (10a).

One side of the guide member (98a) may be disposed adjacent to the first reflector (32) of the first optical system (30) so that air flowing from the heat dissipation fan (96) flows through a flow path formed between the one side of the guide member (98a) and the first reflector (32) of the first optical system (30) . The other side of the guide member (98a) may be formed toward the opening (12a) from the guide member (98a). Since the other side of the guide member (98a) is formed toward the opening (12a), air introduced to the one side of the guide member (98a) may be configured to flow toward the opening (12a).

The guide member (98a) may include a flow path forming part (98b) concavely formed on the inner side thereof. The flow path forming part (98b) may include a plurality of concave parts (98c). The concave parts (98c) formed in the flow path forming part (98b) may be formed such that a lower concave part has a larger width in the width direction than an upper concave part. Through this configuration, friction by air flowing inside the housing can be minimized, and occurrence of a vortex in the air flow can be prevented. Through this, heat dissipation efficiency can be improved.

The flow path guide (98) may include a partition part (99). The partition part (99) may also be a component of the intraoral scanner (2). The partition part (99) may be configured to prevent external air or foreign substances introduced from the opening (12a) from being transferred to the optical module (M). While light moves through the opening (12a) when the intraoral scanner (2) is inserted into the oral cavity of a patient, foreign substances or moisture in the oral cavity may be introduced into the intraoral scanner (2) through the opening (12a). To prevent this, the partition part (99) may prevent air or foreign substances introduced from the outside from moving to the optical module (M).

The partition part (99) may be formed of a transparent material so that light moving along the first and second optical paths (L1, L2) can be transmitted, and may be formed so that the optical module (M) is sealed from the opening (12a). The partition part (99) may be formed in the shape of a transparent plate. The partition part (99) may be configured to block the air flow while allowing light to be transmitted along the first and second optical paths (L1, L2).

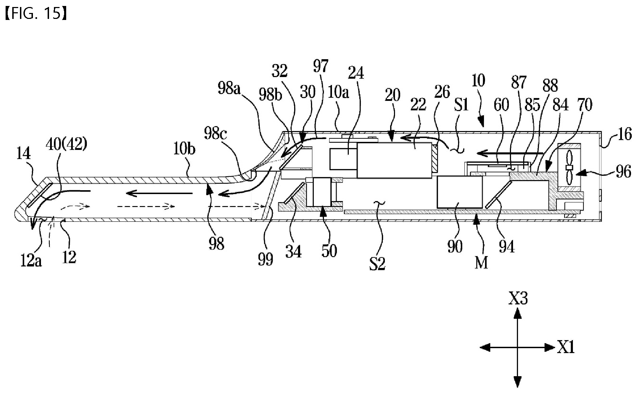

The partition part (99) may be formed to be inclined with respect to the first optical path (L1). Specifically, as shown in FIG. 15, it may be formed to be inclined toward the first optical system (30) relative to being perpendicular to the first optical path (L1). Through this configuration, moisture that may be formed on the partition part (99) may flow down along the surface, and may serve to guide the air flow from the heat dissipation fan (96).

The partition part (99) may be disposed in front of the first optical system (30). The partition part (99) may be configured to prevent external air introduced from the opening (12a) from being introduced through a space between the first reflector (32) and the second reflector (34) of the first optical system (30). To this end, the partition part (99) may be disposed to block the space between the first and second reflectors (32, 34). However, the arrangement and shape of the partition part (99) are not limited thereto. For example, the partition part (99) may be formed between the opening (12a) of the housing (10) and the optical module (M). Also, as a component of the optical module (M), the partition part (99) may extend from the optical module (M) and be formed to surround one side of the optical module (M), or may be formed at the opening forming part (12) of the housing (10). It is satisfied if the partition part (99) is a configuration provided so that air, foreign substances, or moisture introduced through the opening (12a) is not transferred to the optical module (M).

The following describes the air flow of an intraoral scanner according to another embodiment of the present disclosure. In the description, redundant explanations for the same configurations will be omitted.

In the present embodiment, the sensor guide surrounding the circumference of the image sensor unit (60) may be omitted.

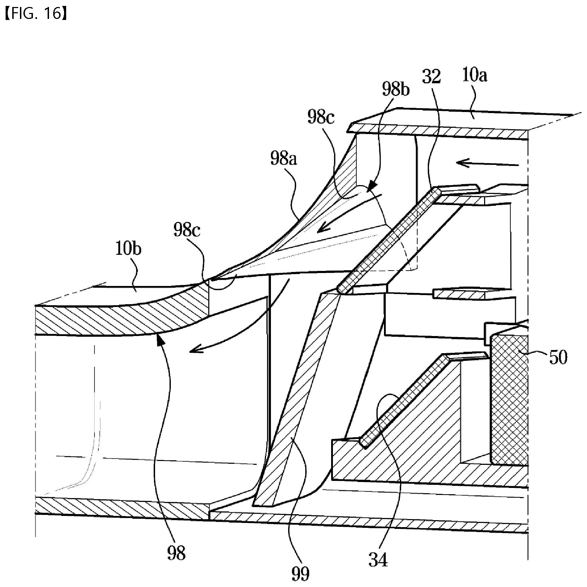

FIG. 16 is a view showing the air flow of an intraoral scanner according to another embodiment of the present disclosure.

The intraoral scanner (2) may form first and second air flow paths (97a, 97b). The first and second air flow paths may be partitioned by the frame (70). The first air flow path (97a) is an air flow path flowing above the inner frame (72), and the second air flow path (97b) may mean an air flow path flowing below the inner frame (72) through the sensor hole (86). The second air flow path (97b) flows below the inner frame (72) through the sensor hole (86) and may merge with the first air flow path (97a) through a space between the light source unit (20) and the first optical system (30). The heat dissipation fan (96) causes air to flow through the first air flow path (97a), thereby dissipating heat generated from the light source unit (20) and the image sensor unit (60) located above the inner frame (72). Also, the heat dissipation fan (96) causes air to flow through the second air flow path (97b), thereby dissipating heat generated from the lens assembly (90) and the third optical system (50). In addition, the heat dissipation fan (96) may dissipate heat from the lower portion of the light source unit (20) installed on the inner frame (72) through the second air flow path (97b).

The heat dissipation fan (96) may cause air to flow into the separation space (87).

The image sensor unit (60) is disposed parallel to the air flow direction of the heat dissipation fan (96), and the image sensor unit (60) is configured to form the separation space (87) spaced apart from the sensor frame (84) by a predetermined distance. Through this, the heat dissipation fan (96) causes air to flow into the separation space (87), thereby efficiently dissipating heat from the upper and lower surfaces of the image sensor unit (60).

In the above, specific embodiments have been illustrated and described. However, the present disclosure is not limited to the above-described embodiments, and various changes and modifications can be made by those skilled in the art without departing from the gist of the technical idea of the invention described in the claims below.

## Claims

1. An intraoral scanner comprising:
a light source unit configured to emit light;
a first optical system configured to reflect light emitted from the light source unit to be moved along a first optical path;
a second optical system configured to reflect light reflected from the first optical system toward an object; and
a third optical system forming a pair of second optical paths with the second optical system, the third optical system being configured to reflect light, which is reflected from the object to the second optical system and moved along the pair of second optical paths, to an image sensor unit,
wherein the third optical system includes:
a pair of first reflectors forming the pair of second optical paths;
a pair of second reflectors configured to reflect light reflected from the pair of first reflectors to the image sensor unit; and
a spacing frame configured to support the pair of first and second reflectors in a state of being spaced apart.

2. The intraoral scanner according to claim 1,
wherein the pair of first and second reflectors are configured to be respectively seated on the spacing frame.

3. The intraoral scanner according to claim 1,
wherein the pair of first and second reflectors each include a pair of first and second reflecting surfaces that are configured to at least partially reflect light and are in contact with the spacing frame.

4. The intraoral scanner according to claim 3,
wherein the spacing frame includes first and second spacing parts on an outside and inside thereof, respectively, on which the first and second reflecting surfaces are respectively supported.

5. The intraoral scanner according to claim 4,
wherein the first spacing part includes a first outer support part and a first inner support part on which the first and second reflecting surfaces are respectively supported;
the second spacing part includes a second outer support part and a second inner support part on which the first and second reflecting surfaces are respectively supported; and
the first outer support part, the first inner support part, the second outer support part, and the second inner support part are configured to be spaced apart from each other by a predetermined angle.

6. The intraoral scanner according to claim 5,
wherein the first inner support part and the second inner support part are formed to be spaced apart at an angle larger than an angle formed between the first outer support part and the second outer support part.

7. The intraoral scanner according to claim 5,
wherein centers of the first outer support part and the second outer support part are disposed on the same line as centers of the first inner support part and the second inner support part.

8. The intraoral scanner according to claim 1,
wherein the spacing frame includes an upper spacing part and a lower spacing part respectively supporting an upper portion and a lower portion of the pair of first and second reflectors.

9. The intraoral scanner according to claim 8,
wherein the upper spacing part and the lower spacing part are configured to be spaced apart from each other by a predetermined distance.

10. The intraoral scanner according to claim 8,
wherein the pair of first and second reflectors, together with the upper spacing part and the lower spacing part, form a light passage through which light reflected by the third optical system passes.

11. The intraoral scanner according to claim 1,
further comprising:
a lens assembly having at least one lens and disposed on a third optical path formed between the pair of second reflectors and the image sensor unit; and
a support frame on which the lens assembly is seated.

12. The intraoral scanner according to claim 11,
wherein the support frame includes first and second support surfaces configured to support symmetrical portions of the lens assembly in a width direction while supporting the lens assembly.

13. The intraoral scanner according to claim 12,
wherein the first and second support surfaces are configured to spread at a predetermined angle.

14. The intraoral scanner according to claim 11,
wherein the support frame is integrally formed with the spacing frame.

15. The intraoral scanner according to claim 1, further comprising:
a sensor frame on which the image sensor unit is disposed; and
a connecting member configured to connect the image sensor unit to the sensor frame.

16. The intraoral scanner according to claim 15,
wherein a plurality of connecting members are provided; and
the plurality of connecting members are disposed along a circumference of the image sensor unit and configured such that the image sensor unit is supported by the sensor frame.

17. The intraoral scanner according to claim 16,
wherein the plurality of connecting members connect the image sensor unit to be spaced apart from the sensor frame so as to form a separation space between the image sensor unit and the sensor frame.

18. The intraoral scanner according to claim 16,
wherein the plurality of connecting members are configured to independently adjust a position of the image sensor unit with respect to the sensor frame.

19. The intraoral scanner according to claim 18,
wherein the position of the image sensor unit includes at least one of an angle and a spacing distance with respect to the sensor frame.
